(19) Europäisches Patentamt
European Patent Office
Office européen des brevets

(11) Veröffentlichungsnummer: **0 667 154 A1**

# (12) EUROPÄISCHE PATENTANMELDUNG

(21) Anmeldenummer: **95100817.6**

(22) Anmeldetag: **21.01.95**

(51) Int. Cl.[6]: **A61K 31/35**, A61K 31/335, A61K 47/22

(30) Priorität: **24.01.94 DE 4401902**

(43) Veröffentlichungstag der Anmeldung: **16.08.95 Patentblatt 95/33**

(84) Benannte Vertragsstaaten: **AT BE CH DE DK ES FR GB IE IT LI NL PT SE**

(71) Anmelder: **MADAUS Aktiengesellschaft**
**Ostmerheimer Strasse 198**
**D-51109 Köln (DE)**

(72) Erfinder: **Lentzen, Hans, PD Dr. rer. nat.**
**Heinrich-Heine-Weg 15**
**D-51503 Rösrath (DE)**
Erfinder: **Mengs, Ulrich, Dr.**
**Am Schmerbroich 69**
**D-53757 St. Augustin 1 (DE)**
Erfinder: **Odenthal, Karl-Peter, Dr.**
**Westfeldstrasse 17**
**D-41517 Grevenbroich (DE)**
Erfinder: **Stolte, Hilmar, Prof. Dr. med.**
**Kaiserstrasse 15**
**D-32545 Bad Oeynhausen (DE)**

## (54) Verwendung von Flavolignanen als Adjuvans in der Tumortherapie.

(57) Die Erfindung betrifft die Verwendung von Flavolignanen als Adjuvans bei der chemotherapeutischen Tumorbehandlung oder zum Schutz der Zellen im Urogenitalbereich.

EP 0 667 154 A1

Die Erfindung betrifft die Verwendung eines Flavolignans als Adjuvans bei der chemotherapeutischen Tumorbehandlung oder zum Schutz der Zellen im Urogenitalbereich.

Flavolignane sind pharmakologisch wirksame Polyhydroxyphenylchromanonverbindungen, die chemisch an Coniferyalkohol gebunden sind und deren bekanntester Vertreter das Silymarin ist. Silymarin ist ein Gemisch von Polyhydroxyphenolchromanonen, das durch Extraktion von Früchten der Mariendistel (Silybum marianum Gaertn.) erhältlich ist, siehe beispielsweise DE-A-19 23 082. Die Bestandteile des Silymaringemisches sind Silibinin, Silidianin und Silichristin, vgl. DE-A-1 923 082 sowie Tetrahedron Letters 25, 2911, 1968; Tetrahedron Letters 31, 2675 - 2678, 1970; und Tetrahedron Letters 22, 1895 - 1899, 1971. Der wichtigste Bestandteil ist Silibinin, das die folgende Strukturformel besitzt:

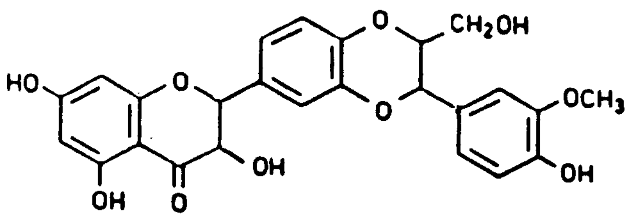

Silymarin bzw. Silibinin sind wertvolle Lebertherapeutika, siehe z. B. DE-A-19 23 082 und DE-A-35 37 656.

Überraschenderweise wurde nun gefunden, daß Silymarin oder die Einzelkomponenten davon als Adjuvans in der Tumortherapie und zum Schutz der Zellen im Urogenitalbereich brauchbar sind.

Gegenstand der vorliegenden Erfindung ist daher die Verwendung mindestens eines Flavolignans als Adjuvans bei der chemotherapeutischen Behandlung von Tumorerkrankungen oder zum Schutz der Zellen im Urogenitalbereich.

Vorzugsweise verwendet man zu diesem Zweck Silymarin oder eine oder mehrere Einzelkomponenten davon. Als besonders brauchbar hat sich dabei Silibinin, insbesondere in Form seines Dihemisuccinatsalzes, erwiesen.

Weiter hat sich gezeigt, daß die erwähnten Flavolignane einen ausgeprägten Schutz vor den Schädigungen bieten, die durch ein Tumortherapeutikum regelmäßig hervorgerufen werden. Die erfindungsgemäß zur Anwendung kommenden Verbindungen sind daher als Adjuvans bei der Tumorbehandlung mit Chemotherapeutika, z. B. Cyclophosphamid, Methotrexat, Vincristin, Vinblastin, Doxorubicin, Bleomycin, Mitomycin, Etoposid, Teniposid, insbesondere Carboplatin, Cisplatin oder Adriamycin und die Derivate davon, brauchbar.

Es hat sich überraschenderweise gezeigt, daß die antitumorale Wirkung von Chemotherapeutika durch die erfindungsgemäß zur Anwendung kommenden Verbindungen nicht beeinflußt wird.

Bekanntlich steigern die Flavolignane die Proteinbiosynthese in Körperzellen und fördern dadurch deren Regeneration. Überraschenderweise wurde nun gefunden, daß dieser Effekt selektiv ausgeübt wird, das heißt, die Flavolignane steigern die Proteinbiosynthese nur in gesunden, intakten Zellen, nicht aber in Tumorzellen. Die Proliferation der Tumorzellen wird somit nicht gefördert, so daß kein schnelleres Wachstum der Tumorzellen erfolgt.

Weiter haben tierexperimentelle Untersuchungen an Ratten ergeben, daß durch Verabreichung der erfindungsgemäß zur Anwendung kommenden Verbindungen die Nephrotoxizität von Zytostatika deutlich gemindert werden kann. Es wurde festgestellt, daß die Flavolignane einen weitgehenden Schutz des glomerulären und tubulären Apparates bieten, so daß ein Schutz des Nierenparenchyms erreicht wird.

Die Flavolignane sind daher zur begleitenden Behandlung in der Tumortherapie brauchbar, weil sie einerseits die Nephrotoxizität der eingesetzten Zytostatika wesentlich mindern, und andererseits die Wirkung der Zytostatika nicht beeinträchtigen und die Proliferation der Tumorzellen nicht fördern.

Weiter wurde gefunden, daß die Flavolignane insbesondere zum Schutz vor nephrotischen Schädigungen und zum Schutz des tubulären und/oder glomerulären Apparates brauchbar sind. Die nephroprotektive Wirkung der Flavolignane ist nicht nur bei Nierenschädigungen zu beobachten, die durch Zytostatika hervorgerufen werden, sondern sie erstreckt sich auf Nierenschädigungen jeder Genese, z. B. auf die durch toxische Wirkung von Schwermetallen, insbesondere Quecksilber- oder Bleiverbindungen, hervorgerufenen Schädigungen, auf ischämische Schädigungen, auf entzündliche Schädigungen, d. h. Nephritiden jeglicher Ursache, u. a. bakteriellen und viralen Ursprungs, sowie auf die bereits erwähnten, durch Zytostatika hervorgerufenen Schädigungen. Die nephroprotektive Wirkung der Flavolignane ist als Schutzwirkung vor den erwähnten Noxen zu verstehen, wobei die Flavolignane selbst keine Nierenschäden verursachen.

Gemäß einer vorteilhaften Ausführungsform kommen die Flavolignane zur Anwendung in Kombination mit mindestens einer Alkalimetallhydrogencitrat-Zusammensetzung oder -Verbindung oder eines Hydrates davon mit der molaren Zusammensetzung K:Na:H:($C_6H_5O_7$) = w:x:y:z

worin w für eine ganze Zahl von 0 bis 15; x für eine ganze Zahl von 0 bis 15; y für eine ganze Zahl von 0 bis 3; z für eine ganze Zahl von 1 bis 5 stehen; wobei

w + x + y = 3; 6; 9; 12 oder 15 entsprechend z = 1; 2; 3; 4 oder 5 und w + x = 1 bis 15 sind, wobei w = 6; x = 6; y = 3 und z = 5 bevorzugt sind. Diese Citrate sind in der DE-A-43 28 577 beschrieben. Durch die Anwendung der Citrate wird der nephroprotektive Effekt der Flavolignane noch verstärkt.

Die erfindungsgemäß zur Anwendung kommenden Verbindungen werden üblicherweise in Form eines pharmazeutischen Mittels verabreicht, das übliche Hilfs- und Zusatzstoffe enthält. Die Mittel können peroral, z.B. in Form von Tabletten, Dragees, Kapseln, Liquidum oder rektal, durch Suppositorien, oder parental, z. B. intravenös, verabreicht werden. Silymarin wird oral verabreicht. Silibinin kann oral, z. B. in Kombination mit oder als Komplex mit Cyclodextrin oder Phospholipiden, wie Lecithin, oder als Methylglucamin- oder Dihemisuccinatsalz verabreicht werden. Die Salze werden auch zur parenteralen Verabreichung eingesetzt. Die erwähnten Kombinationen, Komplexe und Salze sind beschrieben in EP-A-422 497, EP-A-209 037, EP-A-209 038, DE-A-34 42 639 und DE-A-23 02 593.

Die erfindungsgemäß zur Anwendung kommenden Verbindungen können prophylaktisch oder zur Behandlung von bereits eingetretenen Schädigungen verabreicht werden. Als Adjuvans in der Tumortherapie können sie vor, während oder nach Verabreichung des Tumorchemotherapeutikums gegeben werden. Die Dosierung richtet sich nach den Umständen des Einzelfalls, sie liegt im allgemeinen im Bereich von 200 bis 500 mg/Tag beim Mensch.

Die nachfolgenden Versuche erläutern die erfindungsgemäße Verwendung von Silymarin oder der Einzelkomponenten davon.

Es zeigen die Figuren 1 bis 7 die Ergebnisse bei der Cisplatin (CP)-Nephrose:

Figur 1 - Verlauf des Körpergewichtes
Figur 2 - Kreatinin-Clearance
Figur 3 - Proteinurie
Figur 4 - Fibronectinaussscheidung pro 24 Stunden
Figur 5 - Ausscheidung des Enzyms N-Acetyl-$\beta$-D-Glucosaminidase im Urin
Figur 6 - Ausscheidung des Enzyms Alanin-Aminopeptidase (AAP) im Urin
Figur 7 - Fraktionelle Magnesiumausscheidung

Die Figuren 8 bis 11 zeigen die Ergebnisse bei der Adriamycin-Nephrose:

Figur 8 - Körpergewichtsverlauf
Figur 9 - Proteinausscheidung pro 24 Stunden
Figur 10 - Fibronectinausscheidung pro 24 Stunden
Figur 11 - Anstieg der Plasmaspiegel des Malondialdehyds (MDA). Helle Balken: Unmittelbar vor Behandlung. Gestreifte Balken: 1 Stunde nach Behandlung

Die Figuren 12 bis 16 zeigen die Beeinflussung der DNA- und Proteinsynthese bei der Cisplatinbehandlung:

Figur 12 - die Beeinflussung der DNA-Synthese durch Cisplatin
Figur 13 - die spezifische DNA-Synthese der HepG2-Zellen bei Cisplatinbehandlung; Experiment I
Figur 14 die spezifische Proteinsynthese der HepG2-Zellen bei Cisplatinbehandlung; Experiment I
Figur 15, 16 die spezifische DNA- u. Proteinsynthese bei; Experiment II

1. Nephroprotektive Wirkung von Silibinin bei Cisplatin-Nephrose

Das Zystostatikum Cisplatin (CP) gilt als Modellsubstanz zur Erzeugung von tubulären Nierenschäden. Um den protektiven Einfluß von Silibinin als Silibinin-C-2',3-dihydrogensuccinat, Dinatriumsalz zu untersuchen, wurden weibliche Wistar Ratten jeweils wie folgt behandelt:

- 5 mg/kg KG CP i.v. (n = 12)
- 5 mg/kg KG CP i.v. + 200 mg/kg Silibinin i.v. als Silibinin-C-2',3-dihydrogensuccinat, Dinatriumsalz (1h vor CP-Applikation; n = 11)

Zwei weitere Gruppen erhielten entweder nur Silibinin (n = 10) oder das Vehikel (NaCl; n = 12) und dienten als Kontrolle. Die Ratten aller Gruppen wurden über 21 Tage p.a. regelmäßig auf ihre Nierenfunktionen hin untersucht, wobei sich bei folgenden Parametern eine protektive Wirkung von Silibinin zeigte:

Körpergewichtsverlauf:

Nach der Behandlung mit CP kam es zu einer deutlichen Körpergewichtsabnahme. Bei Vorbehandlung mit Silibinin war der Gewichtsverlust deutlich geringer (Fig. 1).

Kreatinin-Clearance:

Die glomeruläre Filtrationsrate (GFR) wurde über die Kreatinin-Clearance bestimmt.
Die Behandlung mit CP führte zu einem starken Abfall der GFR. Bei Vorbehandlung mit Silibinin blieb die GFR im Normbereich (Fig. 2).

Harnstoff:

Parallel zu den Einschränkungen in der GFR kam es nach Gabe von CP zu einem Anstieg der Harnstoffkonzentration im Serum. Durch die Vorbehandlung mit Silibinin wurde ein Anstieg der Harnstoffwerte im Serum verhindert.

Proteinausscheidung:

Die Behandlung mit CP führte zu einem Anstieg der Proteinurie. Silibinin verhinderte diesen Anstieg (Fig. 3).

Fibronectin:

Die Urinkonzentrationen des Strukturproteins Fibronectin wurden durch die Behandlung mit CP signifikant erhöht. Durch die Vorbehandlung mit Silibinin blieben die Werte gegenüber der Kontrolle unverändert (Fig. 4).

N-Acetyl-$\beta$-D-Glucosaminidase (NAG):

Die Ausscheidung des Enzyms NAG im Harn war nach CP-Gabe gegenüber der NaCl-Gruppe erhöht. Bei den mit Silibinin vorbehandelten Tieren waren die NAG-Werte signifikant niedriger als die der mit CP behandelten Ratten (Fig. 5).

Alanin-Aminopeptidase (AAP):

Es ergab sich ein ähnliches Bild wie bei der NAG. Der CP-induzierte Anstieg im Harn fiel bei Vorbehandlung mit Silibinin signifikant geringer aus (Fig. 6).

Magnesium:

Es kam zu einem signifikanten Anstieg der Magnesium-Ausscheidung bei den CP-behandelten Ratten. In der Silibinin + CP-Gruppe war dieser Effekt nicht festzustellen (Fig. 7).

Histopathologie:

Bei der histologischen Beurteilung fanden sich bei den CP-behandelten Ratten massive Nekrosen der Hierentubuli. Bei zusätzlicher Gabe von Silibinin wurden nur vereinzelt Tubulusepithelzellen mit degenerativen Veränderungen beobachtet.

Bei der durch CP induzierten Nephrose wurden also die nachfolgend genannten Parameter durch Silibinin günstig beeinflußt. Es fanden sich:

- geringerer Körpergewichtsverlust
- keine Verringerung der Kreatinin-Clearance
- kein Anstieg der Harnstoffwerte im Serum
- keine Proteinurie
- geringere Fibronectinurie
- geringere Enzymurie (NAG, AAP)
- kein renaler Magnesiumverlust

- deutlich verminderte histopathologische Veränderungen

2. Nephroprotektive Wirkung von Silibinin bei Adriamycin-Nephrose

Das Zytostatikum Adriamycin (ADR) gilt als Modellsubstanz für die Erzeugung einer glomulär-toxischen Nephropathie. Um den Einfluß von Silibinin als Silibinin-C-2',3-dihydrogensuccinat, Dinatriumsalz (Silibinindihemisuccinat) auf die ADR-Nierentoxizität zu untersuchen, wurden weibliche Wistar Ratten wie folgt behandelt:

- 5 mg/kg ADR i.v. (n = 12)
- 5 mg/kg ADR i.v. + 200 mg/kg Silibinin i.v. als Silibinin-C-2',3-dihydrogensuccinat, Dinatriumsalz (1h vor der ADR-Applikation; n = 12)

Zusätzlich wurde je eine Gruppe nur mit Silibinin (n = 6) oder mit dem Vehikel (NaCl; n = 6) behandelt und diente als Kontrolle.

Die Ratten wurden bis zum 21. Tag hinsichtlich der Nierenfunktionen regelmäßig untersucht. Folgende Parameter wurden durch Silibinin günstig beeinflußt:

Körpergewichtsverlauf:

Die Behandlung mit ADR führte zu einem passageren Gewichtsverlust. Dieser Effekt wurde durch die Behandlung mit Silibinin weitgehend kompensiert (Fig. 8).

Proteinausscheidung:

ADR führte zu einem deutlichen Anstieg der Proteinurie. Die Ratten der Silibinin + ADR-Gruppe wiesen ebenfalls einen Anstieg auf, blieben aber zu verschiedenen Zeitpunkten signifikant unter den Werten der ADR-Gruppe (Fig. 9).

Fibronectin:

Die Fibronectinausscheidung stieg nach ADR-Applikation deutlich an. Dieser Effekt trat bei den mit Silibinin vorbehandelten Ratten verzögert auf (Fig. 10).

Malondialdehyd:

Nach Zufuhr von ADR kam es in allen Gruppen zu einem signifikanten Anstieg des Plasmaspiegels für Malondialdehyd. Dieser war jedoch in der zusätzlich mit Silibinin behandelten Gruppe deutlich geringer (Fig. 11).

Bei der durch ADR induzierten Nephrose konnten also durch die Vorbehandlung mit Silibinin nachfolgende Parameter günstig beeinflußt werden. Es fanden sich:

- geringerer Körpergewichtsverlust
- geringere Proteinurie
- geringere Fibronectinurie
- geringerer Plasmaspiegel für Malondialdehyd

3. Nephroprotektion durch Silibinin bei Ratten mit toxischer Nephrose am Beispiel von Quecksilberchlorid

Um die protektive Wirkung von Silibinin (Silibinin-C-2',3-dihydrogensuccinat, Dinatriumsalz) bei der Quecksilberchlorid-Nephropathie zu untersuchen, wurden weibliche Wistar Ratten wie folgt behandelt:

- 0,5 mg/kg $HgCl_2$ i.p. (n = 10)
- 0,5 mg/kg $HgCl_2$ i.p. + 200 mg/kg Silibinin i.v. (30 Minuten vor der $HgCl_2$-Applikation; n = 10)

Vor der Behandlung und am 2. Tag p.a. wurden Blut- und Urinuntersuchungen durchgeführt. Am 3. Tag p.a. wurden die Tiere seziert und die Nieren histologisch untersucht. Dabei ergab sich bei folgenden Parametern eine protektive Wirkung von Silibinin:

Kreatinin:

Durch die Verabreichung von $HgCl_2$ wurden die Kreatininwerte im Plasma deutlich erhöht. Bei den mit Silibinin vorbehandelten Ratten fiel der Anstieg geringer aus (siehe die nachfolgende Tab. 1).

Malatdehydrogenase (MDH):

Die Behandlung mit $HgCl_2$ führte zu einem Anstieg der MDH im Harn. In der Silibinin-Gruppe war dieser deutlich geringer ausgeprägt (siehe die nachfolgende Tab. 1).

Gammaglutamyltransferase ($\gamma$-GT):

Im Urin der $HgCl_2$-behandelten Tiere ließen sich stark erhöhte $\gamma$-GT-Aktivitäten messen. Bei den mit Silibinin vorbehandelten Tieren war dagegen nur ein geringgradiger Anstieg festzustellen (siehe die nachfolgende Tab. 1).

Lactatdehydrogenase (LDH):

Ähnlich wie bei der MDH und der $\gamma$-GT ließ sich der durch $HgCl_2$ induzierte Enzymanstieg der LDH im Harn durch eine Vorbehandlung mit Silibinin deutlich vermindern (siehe die nachfolgende Tab. 1).

Glucosurie:

Nach Verabreichung von $HgCl_2$ war eine massive Erhöhung der Glukoseausscheidung festzustellen. Bei den mit Silibinin vorbehandelten Ratten waren die Glukosekonzentrationen im Harn signifikant niedriger (siehe die nachfolgende Tab. 1).

Histopathologie:

Bei der histologischen Beurteilung fanden sich in den Nieren der mit $HgCl_2$ behandelten Ratten deutliche Anzeichen einer tubulären Degeneration bis hin zur Nekrose. Die Veränderungen waren bei den mit Silibinin vorbehandelten Ratten deutlich geringer ausgeprägt.

Durch eine Vorbehandlung mit Silibinin konnte somit die Entwicklung einer mit Quecksilberchlorid induzierten toxischen Nephropathie günstig beeinflußt werden. Es fanden sich:

- verminderter Anstieg des Kreatinin-Plasmaspiegels
- verminderte Enzymurie (MDH, $\gamma$-GT, LDH)
- signifikant verminderte Glukosurie
- histologisch deutlich geringere Tubulusschäden

Tabelle 1

| Einfluß von Silibinin auf die Quecksilber-chlorid-Nephropathie an Ratten (Mittelwert ± Standardabweichung; n = 10) | | | |
|---|---|---|---|
| Parameter | Versuchstag | $HgCl_2$ | $HgCl_2$ + Silibinin |
| Kreatinin im Plasma (µmol/l) | -3<br>2 | 39,8 ± 3,22<br>51,8 ± 15,68 | 37,1 ± 3,41<br>42,7 ± 8,98 |
| MDH im Harn (U/V16h) | -3<br>2 | 0,27 ± 0,085<br>3,96 ± 8,091 | 0,24 ± 0,079<br>1,98 ± 4,863 |
| $\gamma$-GT im Harn (U/V16h) | -3<br>2 | 5,02 ± 3,75<br>11,51 ± 15,996 | 3,75 ± 1,228<br>6,90 ± 5,824 |
| LDH im Harn (U/V16h) | -3<br>2 | 0,071 ± 0,0437<br>1,511 ± 2,3390 | 0,057 ± 0,0477<br>1,055 ± 2,7200 |
| Glukose im Harn (µmol/V16h) | -3<br>2 | 4,23 ± 1,363<br>72,98 ± 86,855 | 3,55 ± 0,494<br>9,76 ± 20,220 |

4. Modulation der Cisplatin-mediierten Cytotoxizität auf HepG2-Humantumorzellen

Wie oben gezeigt, besitzt Silibinin nephroprotektive Ativität gegenüber der Nephrotoxizität von Cisplatin, Adriamycin und Quecksilberchlorid. Bei der Tumortherapie mittels Cytostatika wäre eine Beeinträchtigung der Cytostatikawirkung durch das erfindungsgemäß zur Anwendung kommende Adjuvans ebenso unerwünscht wie die Förderung des Tumorwachstums durch das Adjuvans. Um dies zu untersuchen, wurde die mögliche Modulation der Cisplatin-mediierten Cytotoxizität durch Silibinin an HepG2-Humantumorzellen in vitro untersucht.

HepG2-Tumorzellen sind empfindliche Indikatoren der Cisplatin-mediierten Cytotoxizität und daher als in vitro Modell geeignet, eine Modulation des cytostatischen Effektes von Cisplatin nachzuweisen.

Um den Effekt von Silibinin auf die cytostatische Wirkung von Cisplatin zu untersuchen, wurde das Wachstum von Tumorzellen verfolgt, indem die DNA-Synthese durch Einbau von $^{3}$H-Thymidin in die celluläre DNA gemessen wurde.

Die Gesamtproteinsynthese in der Zelle wurde durch Einbau von $^{3}$H-Leucin gemessen und um bestimmen zu können, ob Silibinin eine unerwünschte stimulierende Aktivität auf die Proteinsynthese der Tumorzelle besitzt.

Das Silibinin kam in Form des Dinatriumsilibinin-Dihemisuccinats (im folgenden kurz als Silibinin bezeichnet) gelöst in Dimethylsulfoxid zur Anwendung. Die Kontrollen wurden durchgeführt unter Verwendung von unbehandelten (=negativen) Kontrollen, Lösungsmittelkontrollen (=1 % Dimethylsulfoxid enthaltendes Medium), Silibinin ohne Cisplatin und Cisplatin ohne Silibinin.

Um festzustellen, ob eine direkte Interaktion zwischen Silibinin und Cisplatin vorliegt, wurde auch eine Co-Inkubation beider Substanzen nach der Silibinin-Vorinkubation vorgesehen.

Nach Beendigung beider Behandlungsperioden wurde entweder die DNA-Synthese oder die Gesamtproteinsynthese bestimmt.

Für beide Endpunkte wurden zwei unabhängige Versuche durchgeführt.

DNA-Synthese

Während der Silibinin-Behandlungsperiode wurden die Zellen mit 5 mCi/ml $^{14}$C-Thymidin markiert, das als Parameter für die Zahl der adhärenten Zellen diente. Die Zellen wurden dann zweimal mit RPMI gewaschen und eine Stunde mit Cisplatin behandelt. Anschließend wurden die Zellen mit RPMI gewaschen, und mit 200 µl Medium, das 2,5 µCi/ml $^{3}$H-Thymidin (0,5 µCi/Kultur) enthielt, gefüttert. Nach einstündiger radioaktiver Markierung wurden die Zellen, wie oben beschrieben, geerntet.

Die eingebaute Menge an $^{3}$H und $^{14}$C wurde durch Flüssigkeitsscintillationszählung bestimmt. Das $^{3}$H/$^{14}$C-Verhältnis wurde berechnet, um die bezüglich der Zellzahl korrigierte DNA-Synthese zu erhalten. Dieses Verhältnis wird als spezifische DNA-Synthese bezeichnet.

Gesamtproteinsynthese

2,5 x $10^5$ HepG2-Zellen/Kultur wurden in 24-Well Platten mit Silibinin und Cisplatin, wie oben für die DNA-Synthese beschrieben, behandelt. Nach Waschen mit RPMI wurden die Zellen mit RPMI gefüttert, das mit 10% FCS supplementiert war. Anschließend wurden sofort 0,5 µCi/ml $^{3}$H-Leucin pro Vertiefung zugegeben, um das neu synthetisierte Zellprotein zu markieren. Nach zweistündiger Markierung wurde das Medium abgesaugt und die Monoschicht wurde mit PBS gewaschen. Der Zellinhalt wurde durch Zugabe von 1 ml 0,1 % Nonidet NP 40 in PBS und 30-minütiges heftiges Schütteln freigesetzt.

200 µl Anteile wurden zur Proteinbestimmung nach Färben mit Biorad-Farbreagens bei 595 nm unter Verwendung eines Spektralphotometers herangezogen. Man überführte die 500 µl Anteile in Zentrifugenröhrchen aus Polypropylen, gab 500 µl eiskalte, 60%-ige Trichloressigsäure (TCA) zu und fällte das Säureunlösliche Material 30 Minuten bei 4°C. Nach 10-minütiger Zentrifugation bei 4000 Upm wurde das Pellet mit 30%iger TCA (4°C) gewaschen und wenigstens 10 Minuten mit 0,5 ml einer 0,3 N NaOH bei 60°C solubilisiert.

Die Lösung wurde mit 0,1 ml einer 0,1 N HCl neutralisiert.

Die eingebaute Menge $^{3}$H-Leucin wurde durch Flüssigkeitsscintillationszählung bestimmt. Der $^{3}$Hdpm/$OD_{595}$-Wert wurde berechnet, um die bezüglich der Zellzahl korrigierte Gesamtproteinsynthese zu erhalten. Dieser Wert wird als spezifische Proteinsynthese bezeichnet.

Die folgenden Konzentrationen wurden für die Versuche ausgewählt:
Silibinin-Vorbehandlung: 50,00 und 300,00 µg/ml
Cisplatin: 1,00; 10,00; 100,00; und 1000,00 µg/ml.

In beiden Experimenten führte die Präinkubation mit Silibinin zu keiner Beeinträchtigung der anhand der DNA-Synthese der HepG2-Zellen-bestimmten cytostatischen Aktivität des Cisplatins.

Die Behandlung mit 50 µg/ml oder 300 µg/ml Silibinin alleine erhöhte weder die DNA-Synthese noch die Proteinsynthese der verwendeten HepG2-Zellen. Dies zeigt, daß Silibinin keine die Tumorzellen aktivierenden Eigenschaften besitzt.

Zusammenfassend ist daher festzustellen, daß Silibinin weder einen unerwünschten inhibierenden Effekt auf die Cytotoxizität des Cisplatins noch eine unerwünschte stimulierende Aktivität auf die von HepG2-Zellen aufweist.

Die erhaltenen Ergebnisse sind in den nachfolgenden Tabellen 2 und 3 und den Figuren 12 - 16 zusammengefaßt.

Tabelle 2: Hauptversuch: DNA-Synthese

| | | Experiment I | | Experiment II | |
|---|---|---|---|---|---|
| SB16-WG779 [µg/ml] | Cisplatin [µg/ml] | $^{3}H$ / $^{14}C$ [1] | % bezogen auf Lösungsmittel kontrolle | $^{3}H$ / $^{14}C$ [1] | % bezogen auf Lösungsmittel-kontrolle |
| - | 0.0 | 2.99[2] | 100 | 2.79[2] | 100 |
| - | 1.0 | 2.17 | 73 | 1.93 | 69 |
| - | 10.0 | 1.54 | 52 | 1.67 | 60 |
| - | 100.0 | 0.43 | 14 | 0.70 | 25 |
| - | 1000.0 | 0.10 | 3 | 0.12 | 4 |
| - | 0.0 | 2.99[2] | 100 | 2.79[2] | 100 |
| 50.0 | 0.0 | 2.48 | 83 | 2.57 | 92 |
| 50.0 | 1.0 | 2.24 | 75 | 2.55 | 91 |
| 50.0 | 10.0 | 1.83 | 61 | 1.79 | 64 |
| 50.0 | 100.0 | 0.39 | 13 | 0.52 | 19 |
| 50.0 | 1000.0 | 0.14 | 5 | 0.13 | 5 |
| - | 0.0 | 2.99[2] | 100 | 2.79[2] | 100 |
| 300.0 | 0.0 | 2.87 | 96 | 2.52 | 90 |
| 300.0 | 1.0 | 2.18 | 73 | 2.20 | 79 |
| 300.0 | 10.0 | 2.17 | 73 | 1.66 | 59 |
| 300.0 | 100.0 | 0.46 | 15 | 0.47 | 17 |
| 300.0 | 1000.0 | 0.06 | 2 | 0.17 | 6 |
| - | 0.0 | 3.66[3] | 100 | 2.89 | 100 |
| 50.0* | 0.0* | 2.69 | 73 | 3.19 | 110 |
| 50.0* | 1.0* | 2.83 | 77 | 2.81 | 97 |
| 50.0* | 10.0* | 3.03 | 83 | 2.01 | 70 |
| 50.0* | 100.0* | 0.68 | 19 | 0.77 | 27 |
| 50.0* | 1000.0* | 0.09 | 2 | 0.15 | 5 |
| - | 0.0 | 3.66[3] | 100 | 2.89 | 100 |
| 300.0* | 0.0* | 3.82 | 104 | ** | ** |
| 300.0* | 1.0* | 2.65 | 72 | 1.36 | 47 |
| 300.0* | 10.0* | 2.38 | 65 | 1.44 | 50 |
| 300.0* | 100.0* | 0.82 | 22 | 0.59 | 20 |
| 300.0* | 1000.0* | 0.11 | 3 | 0.09 | 3 |

1 = Spezifische DNA-Synthese, Mittelwert von 3 Kulturen
2 = Mittelwert von 9 Kulturen
3 = Mittelwert von 6 Kulturen
* = 24 h Vorbehandlung mit SB16-WG779 + 1 h co-Inkubation mit SB16-WG779 und Cisplatin
** = nicht bewertbar

SB16-WG779 = Silibinin-C-2',3-dihydrogensuccinat, Dinatriumsalz (Silibinindihemisuccinat)

Tabelle 3: Hauptversuch: Protein-Synthese

| | | Experiment I | | Experiment II | |
|---|---|---|---|---|---|
| SB16-WG779 [µg/ml] | Cisplatin [µg/ml] | $^3H$ / $OD_{595}$ [1] | % bezogen auf Lösungsmittel-kontrolle | $^3H$ / $OD_{595}$ [1] | % bezogen auf Lösungsmittel-kontrolle |
| - | 0.0 | 546.4[2] | 100 | 679.5[2] | 100 |
| - | 1.0 | 438.5 | 80 | 925.0 | 136 |
| - | 10.0 | 472.8 | 87 | 971.4 | 143 |
| - | 100.0 | 242.5 | 44 | 322.0 | 47 |
| - | 1000.0 | 98.0 | 18 | 134.6 | 20 |
| - | 0.0 | 593.1 | 100 | 549.8 | 100 |
| 50.0 | 0.0 | 1108.6 | 187 | 1171.4 | 213 |
| 50.0 | 1.0 | 601.0 | 101 | 1200.4 | 218 |
| 50.0 | 10.0 | 748.2 | 126 | 1109.3 | 202 |
| 50.0 | 100.0 | 497.4 | 84 | 367.9 | 67 |
| 50.0 | 1000.0 | 118.8 | 20 | 160.5 | 29 |
| - | 0.0 | 1296.9 | 100 | 952.0 | 100 |
| 300.0 | 0.0 | 778.8 | 60 | 824.9 | 87 |
| 300.0 | 1.0 | 905.3 | 69 | 1306.4 | 137 |
| 300.0 | 10.0 | 841.9 | 65 | 1224.7 | 129 |
| 300.0 | 100.0 | 363.7 | 28 | 451.2 | 47 |
| 300.0 | 1000.0 | 123.7 | 10 | 145.9 | 15 |
| - | 0.0 | 1298.1 | 100 | 2008.8 | 100 |
| 50.0* | 0.0* | 852.6 | 66 | 776.2 | 39 |
| 50.0* | 1.0* | 652.1 | 50 | 809.9 | 40 |
| 50.0* | 10.0* | 583.9 | 45 | 1343.1 | 67 |
| 50.0* | 100.0* | 295.3 | 23 | 298.3 | 15 |
| 50.0* | 1000.0* | 118.1 | 9 | 172.2 | 9 |
| - | 0.0 | 688.2 | 100 | 483.4 | 100 |
| 300.0* | 0.0* | 737.2 | 107 | 1427.5 | 295 |
| 300.0* | 1.0* | 1586.9 | 230 | 1509.1 | 312 |
| 300.0* | 10.0* | 680.7 | 99 | 1427.5 | 295 |
| 300.0* | 100.0* | 395.4 | 58 | 295.0 | 61 |
| 300.0* | 1000.0* | 136.9 | 10 | 148.9 | 31 |

1 = Spezifische Protein-Synthese, Mittelwert von 3 Kulturen
2 = Mittelwert von 6 Kulturen
* = 24 h Vorbehandlung mit SB16-WG779 + 1 h co-Inkubation mit SB16-WG779 und Cisplatin

SB16-WG779 = Silibinin-C-2',3-dihydrogensuccinat, Dinatriumsalz (Silibinindihemisuccinat)

## Patentansprüche

1. Verwendung mindestens eines Flavolignans als Adjuvans bei der chemotherapeutischen Tumorbehandlung oder zum Schutz der Zellen im Urogenitalbereich.

2. Verwendung nach Anspruch 1 zur Verminderung der durch ein Tumortherapeutikum bedingten Schädigungen.

3. Verwendung nach Anspruch 1 oder 2 zur Prophylaxe und Behandlung von nephrotischen Schädigungen.

4. Verwendung nach Anspruch 1 oder 2 zum Schutz des tubulären und/oder glomerulären Apparates.

5. Verwendung nach Anspruch 1 oder 2 als Adjuvans zu Cis-Platin oder Adriamycin oder einem Derivat davon.

6. Verwendung nach einem der vorhergehenden Ansprüche, wobei man als Flavolignan Silymarin oder mindestens eine Einzelkomponente davon einsetzt.

7. Verwendung nach Anspruch 6, wobei man Silibinin, insbesondere Silibinin-Dihemisuccinat, einsetzt.

8. Verwendung nach einem der vorhergehenden Ansprüche, wobei das Flavolignan in Kombination mit mindestens einer Alkalimetallcitrat-Zusammensetzung oder Verbindung oder einem Hydrat davon mit der molaren Zusammensetzung

$$K:Na:H:(C_6H_5O_7) = w:x:y:z$$

worin w für eine ganze Zahl von 0 bis 15; x für eine ganze Zahl von 0 bis 15; y für eine ganze Zahl von 0 bis 3; z für eine ganze Zahl von 1 bis 5 stehen; wobei $w + x + y = 3; 6; 9; 12$ oder 15 entsprechend $z = 1; 2; 3; 4$ oder 5 und $w + x = 1$ bis 15 sind.

9. Pharmazeutisches Mittel, enthaltend mindestens ein Tumortherapeutikum und mindestens ein Flavolignan.

10. Mittel nach Anspruch 9, das als Flavolignan Silymarin oder mindestens eine Einzelkomponente davon enthält.

11. Mittel nach Anspruch 10, wobei es sich bei der Einzelkomponente des Silymarins um Silibinin, insbesondere Silibinin-Dihemisuccinat, handelt.

12. Mittel nach einem der Ansprüche 9 bis 11, wobei es sich bei dem Tumortherapeutikum um Cis-Platin oder Adriamycin oder ein Derivat davon handelt.

13. Mittel nach einem der Ansprüche 9 bis 12, das zusätzlich wenigstens eine Alkalimetallcitrat-Zusammensetzung oder -Verbindung wie in Anspruch 8 definiert enthält.

Figur 1 (Körpergewichte)

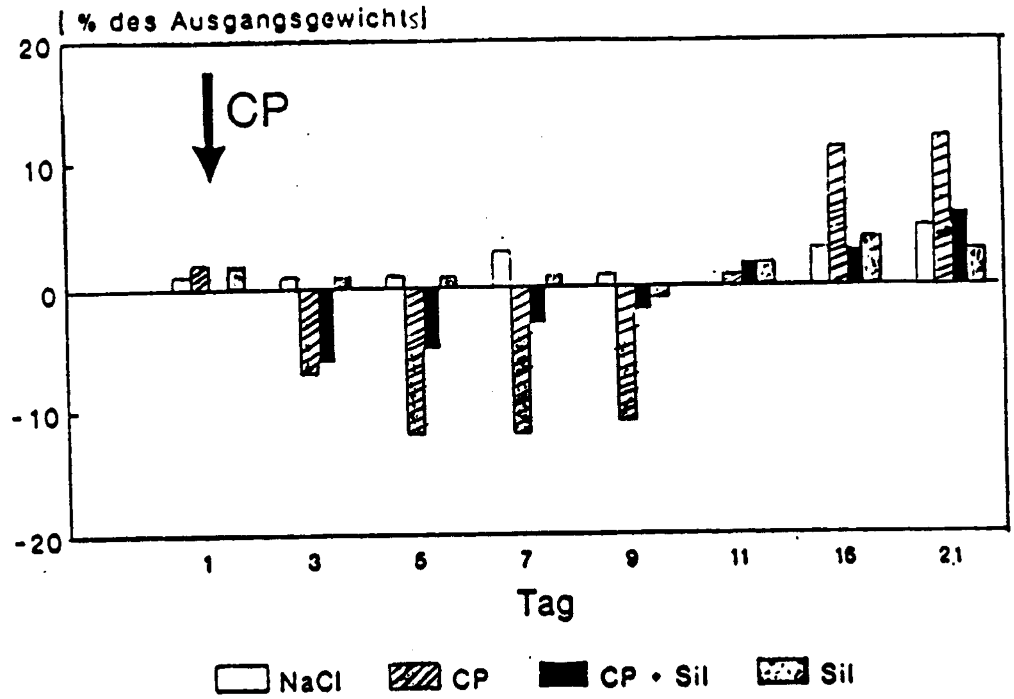


Figur 2 (Kreatinin-Clearance)

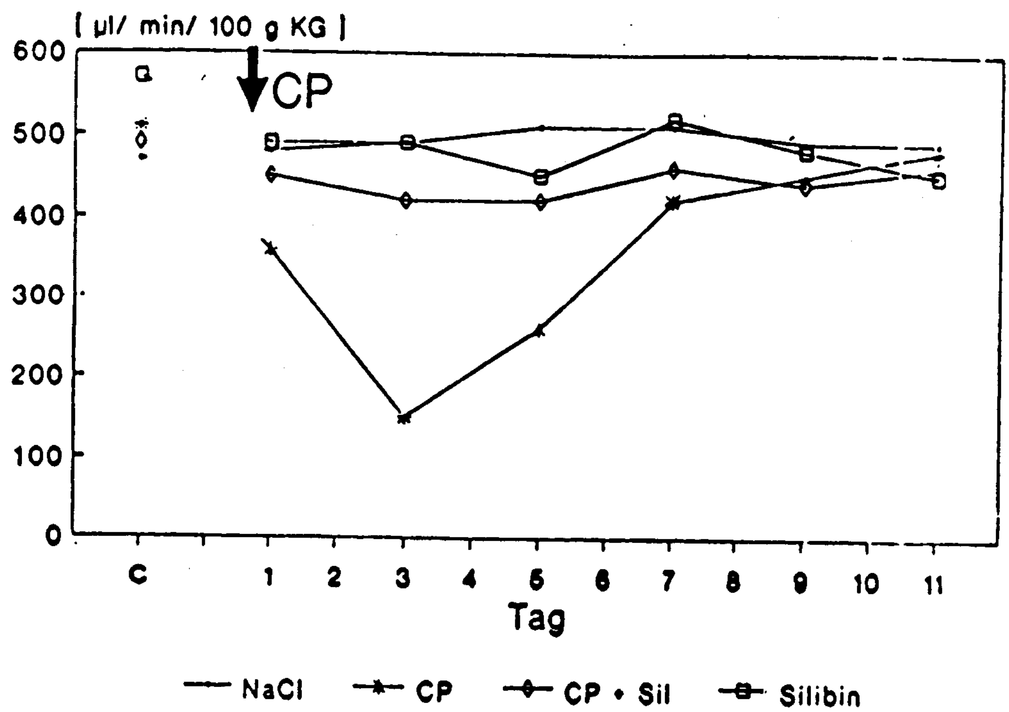


CP = Cisplatin

Figur 3 (Protein)

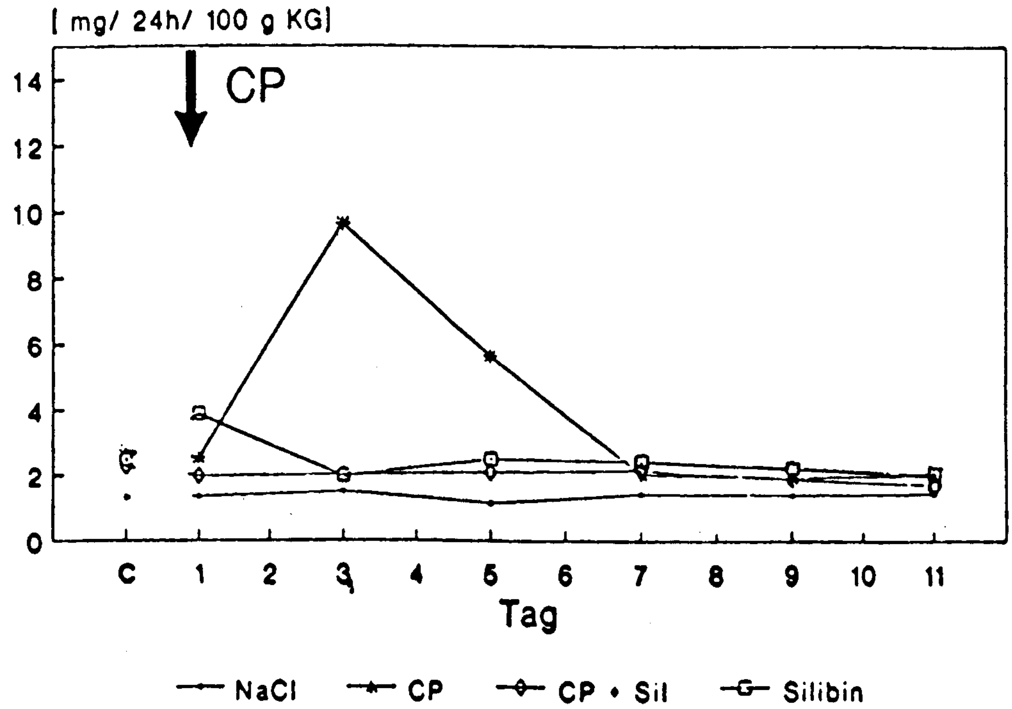


Figur 4 (Fibronectin)

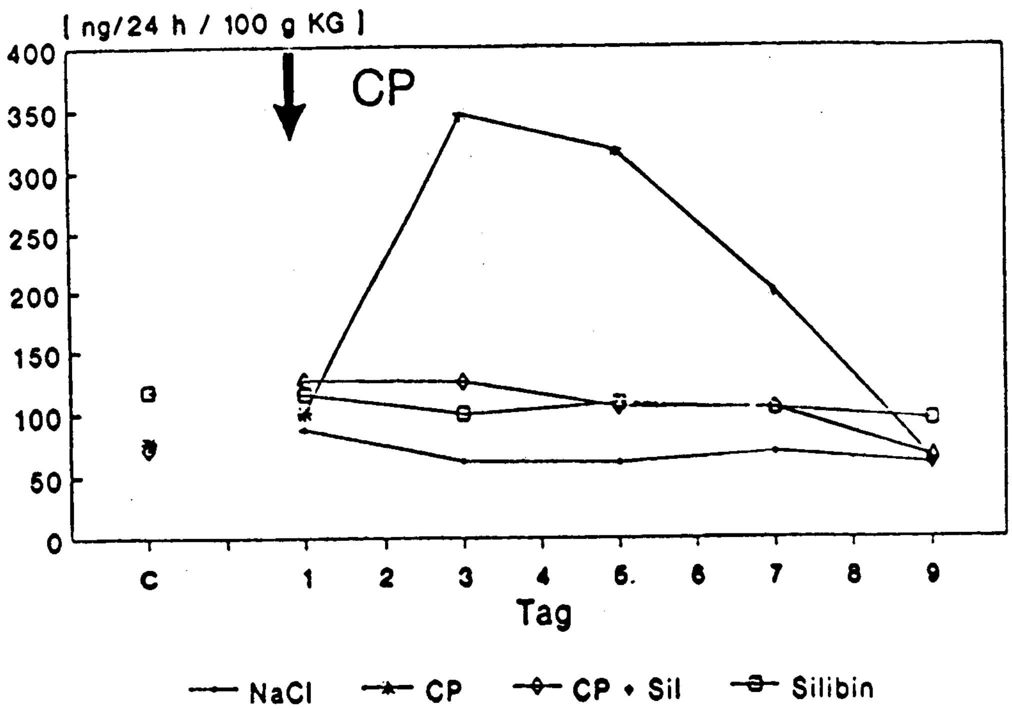

Figur 5 (NAG = N-Acetyl-ß-D-Glucosaminidase)

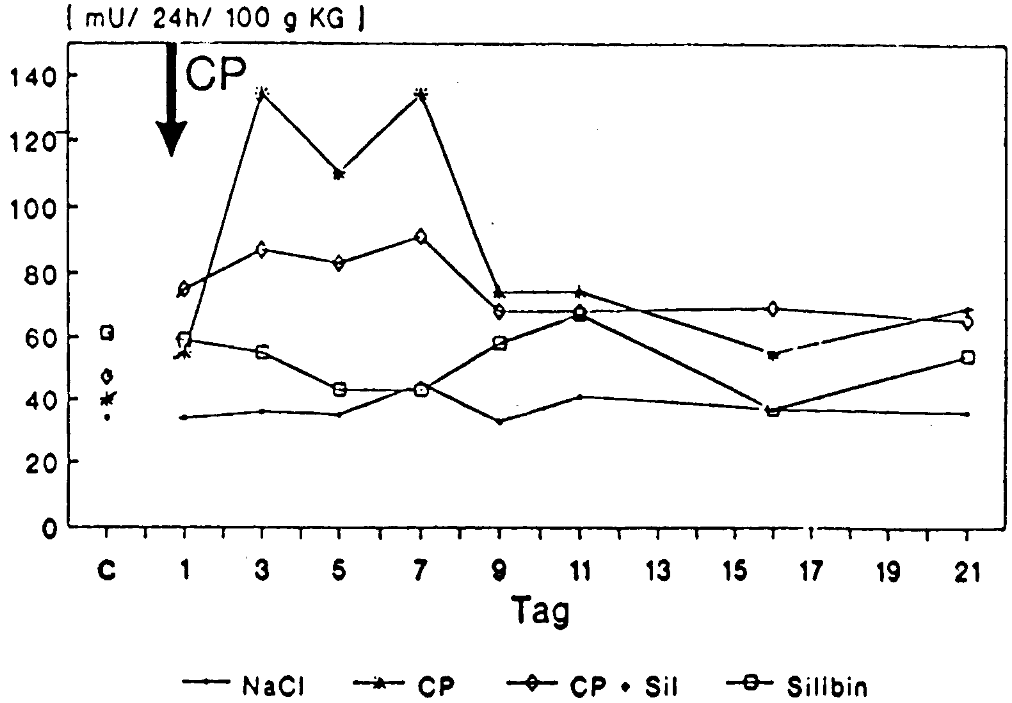


Figur 6 (AAP = Alanin-Aminopepditase)

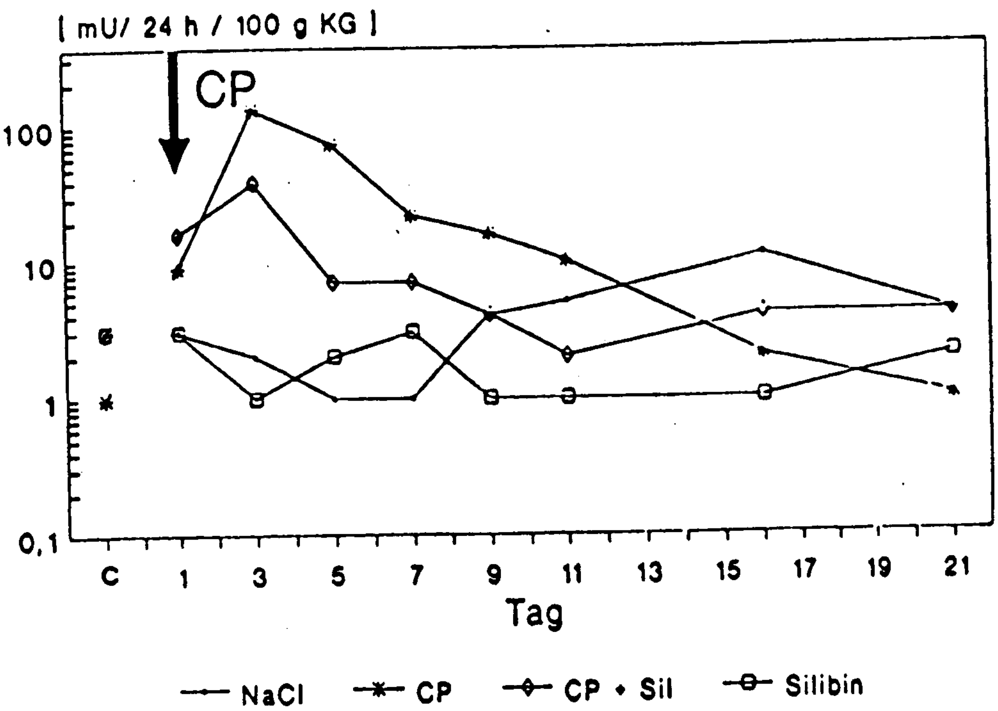

Figur 7 (Magnesium)

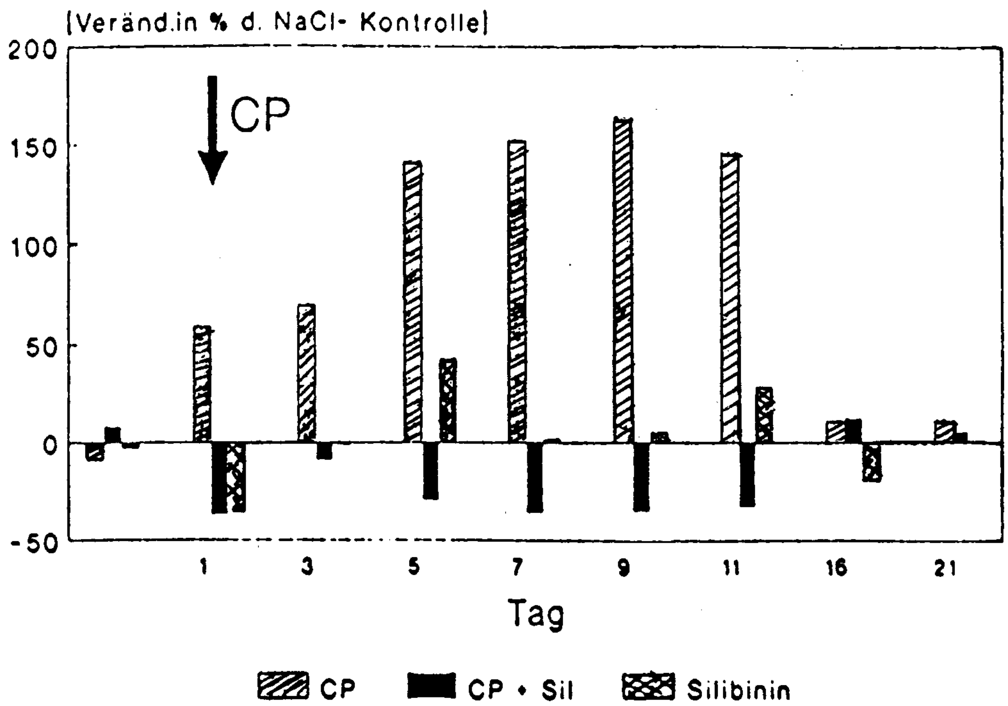


Figur 8

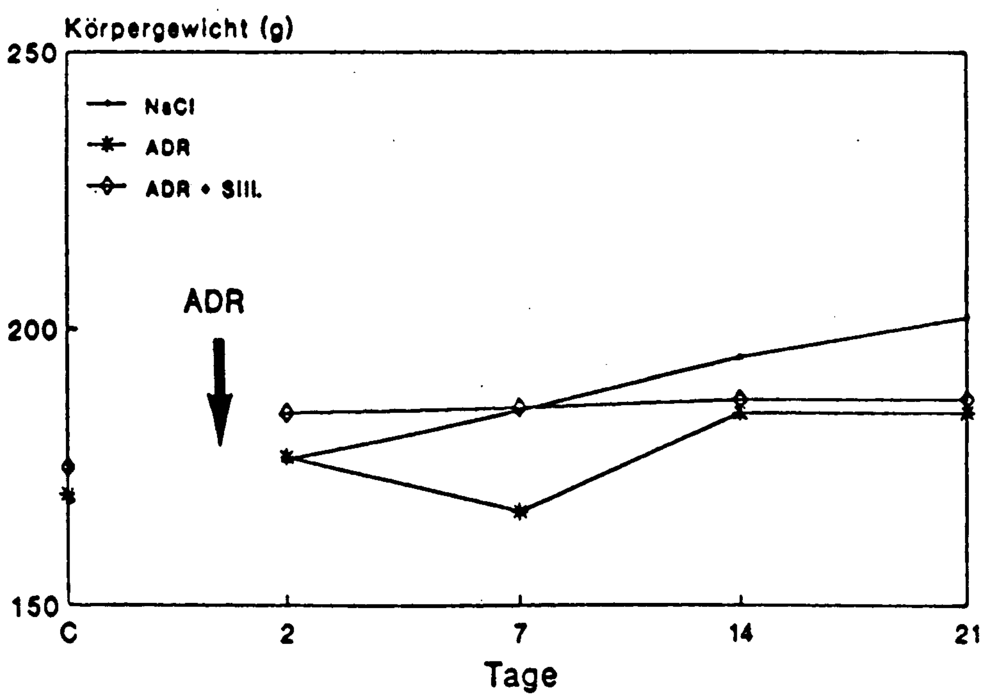


ADR = Adriamycin

Figur 9

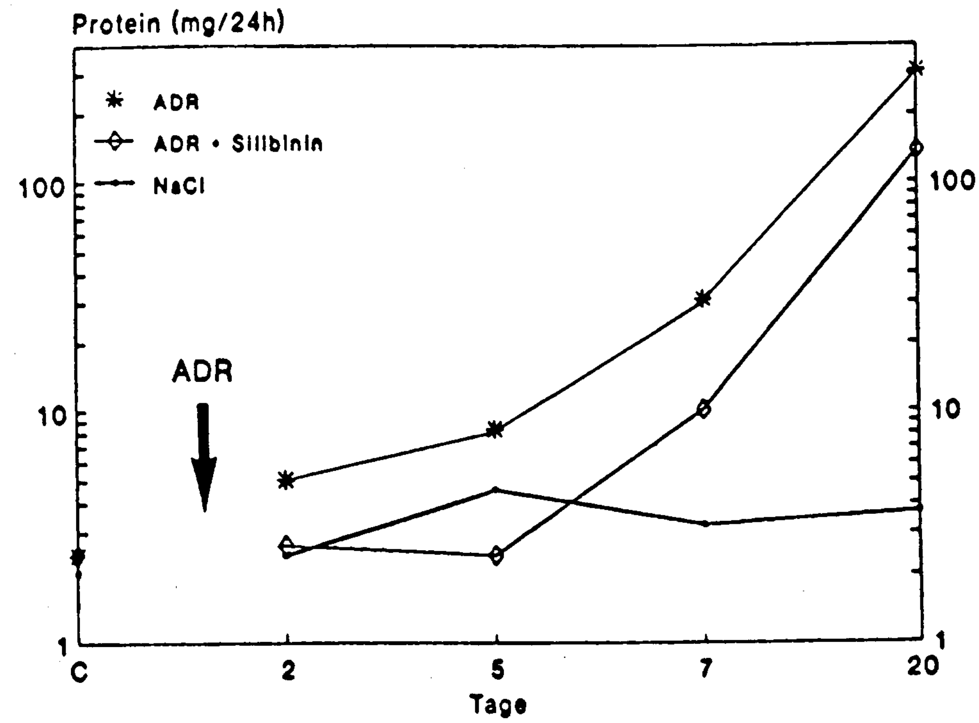


Figur 10

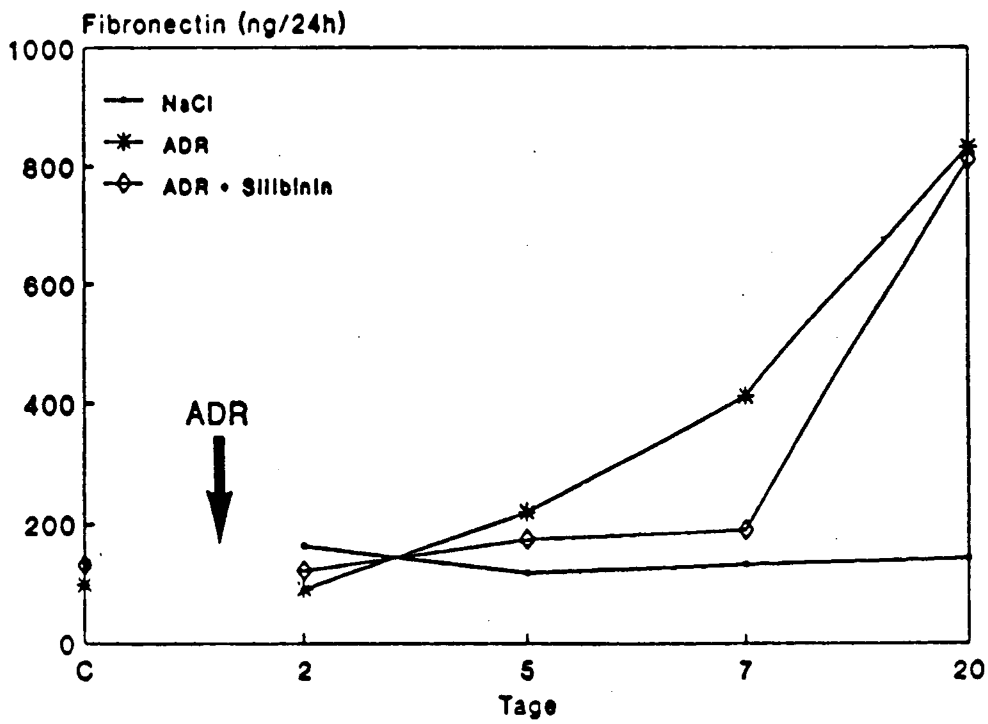

Figur 11 (Plasmaspiegel MDA)

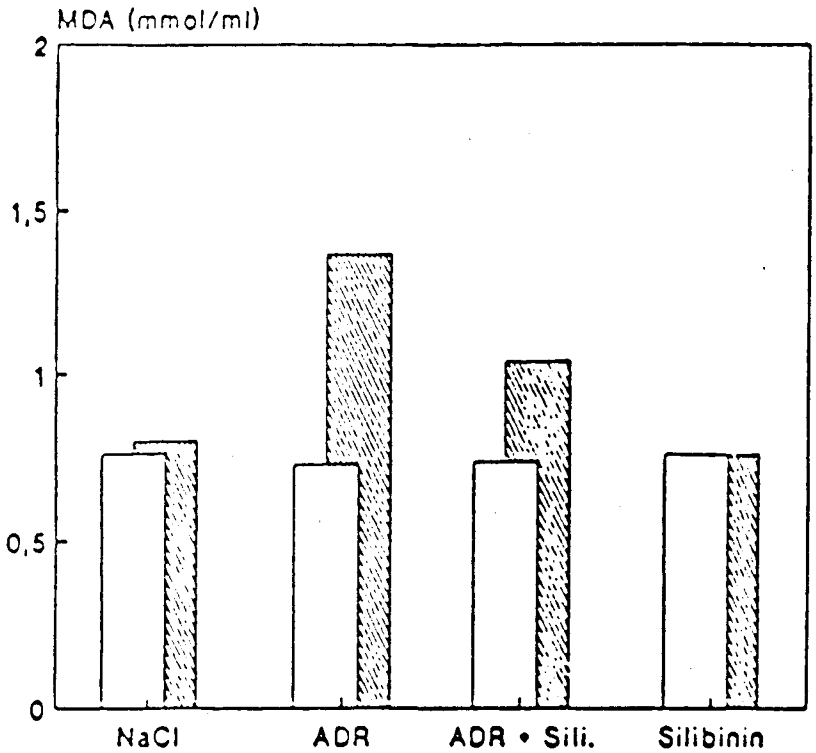


ADR = Adriamycin

MDA = Malondialdehyd

Figur 12

DNA-Synthese (% bezogen auf Lösungsmittelkontrolle)

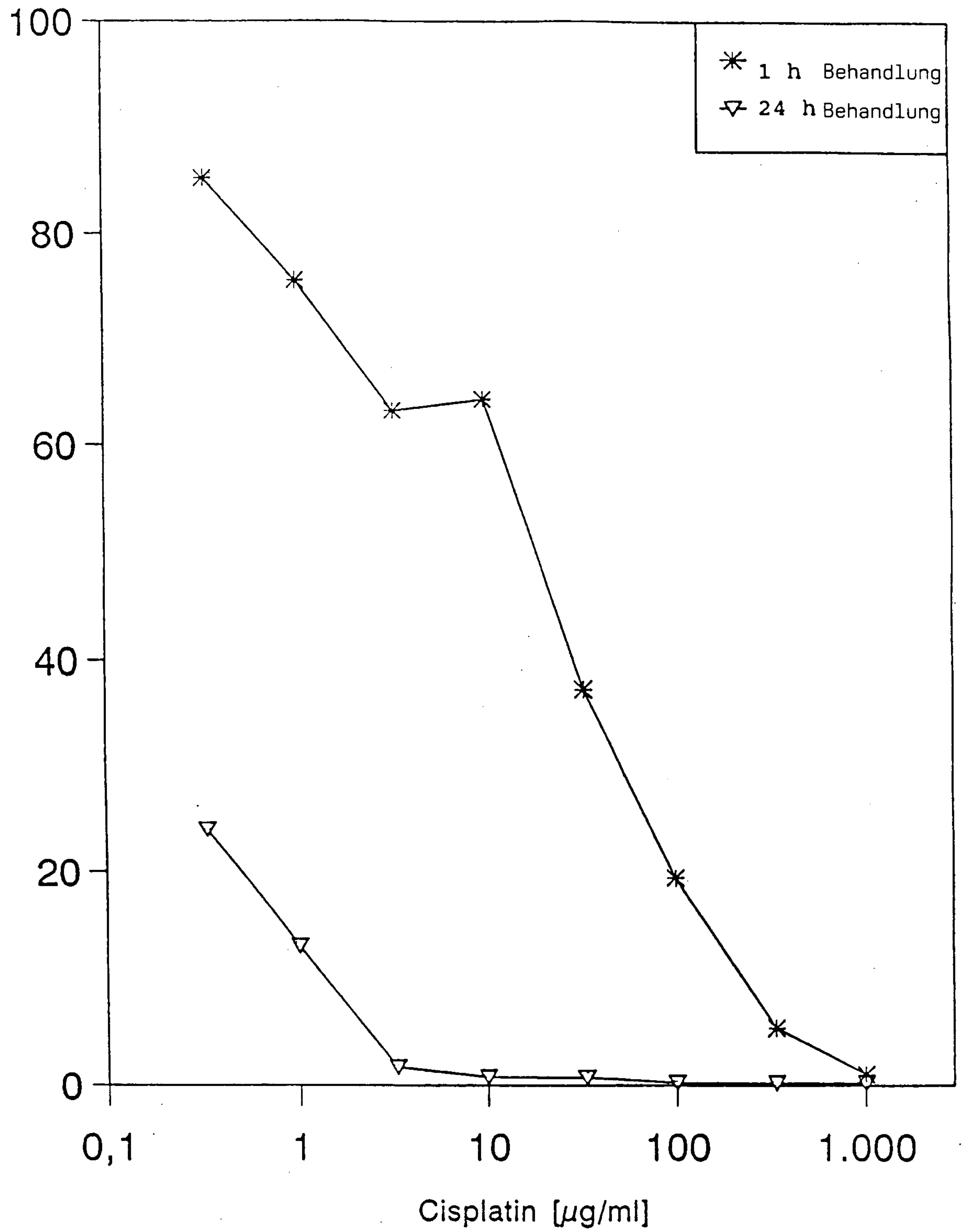

Figur 13
Spezifische DNA-Synthese (% bez. auf Lösungsmittelkontrolle)

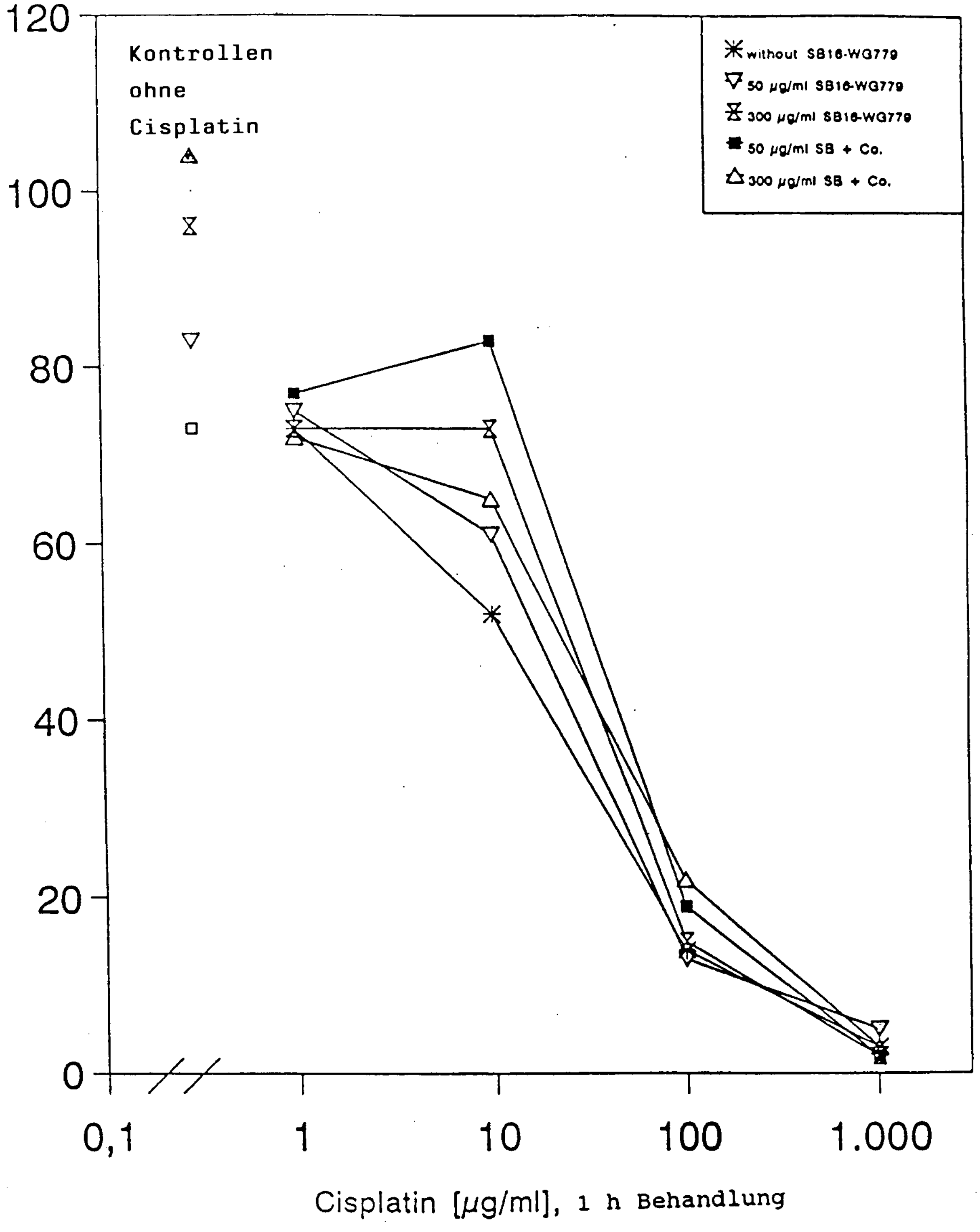


SB = Silibinin als SB16-WG779
SB16-WG779 = Silibinin-C-2',3-dihydrogensuccinat, Dinatriumsalz (Silibinindihemisuccinat)
Co = Coinkubation

Figur 14

Spezifische Protein-Synthese (% bez. auf Lösungsmittelkontrolle)

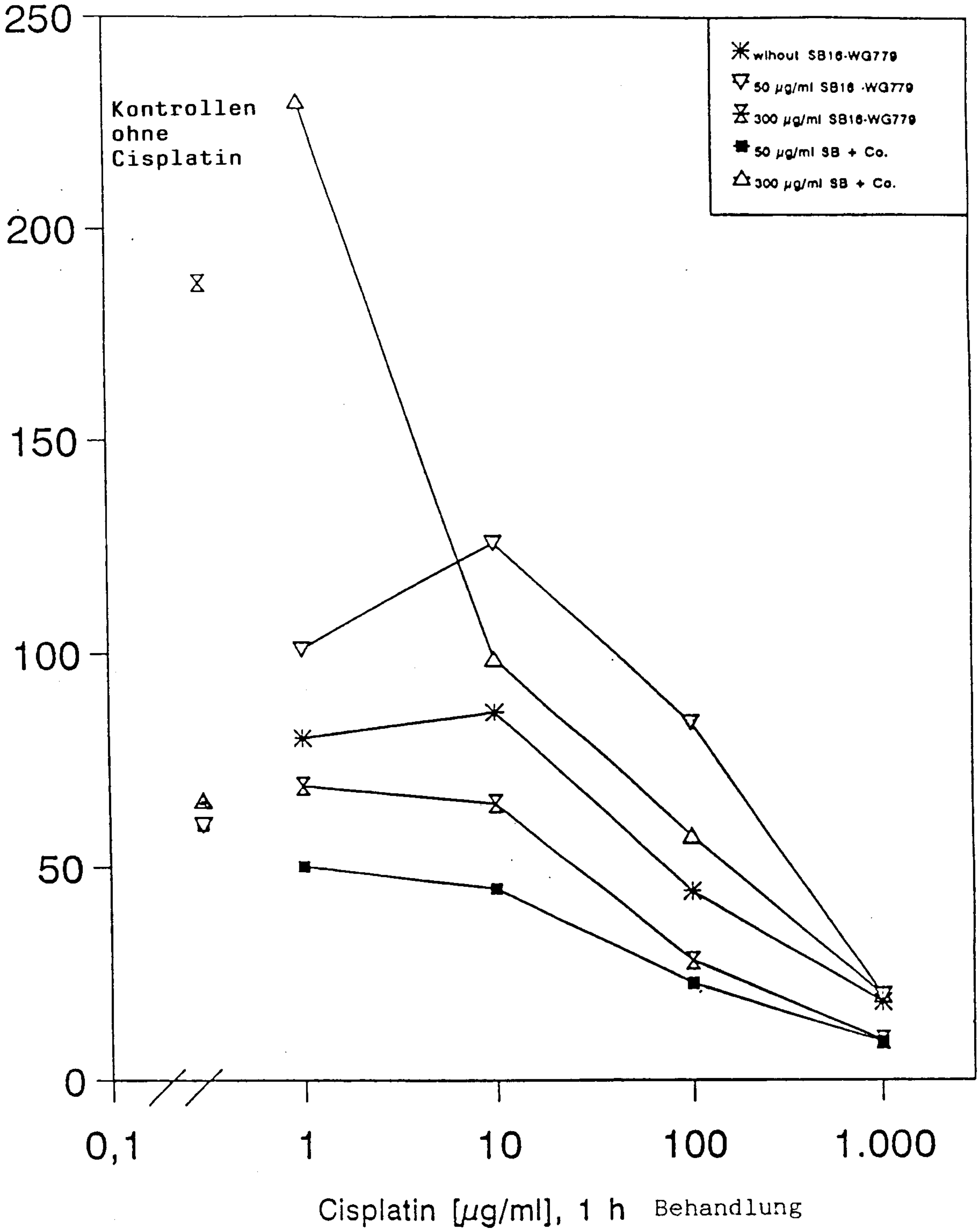


SB = Silibinin als SB16-WG779

SB16-WG779= Silibinin-C-2',3-dihydrogensuccinat, Dinatriumsalz (Silibinindihemisuccinat)

Co = Coinkubation

Figur 15

Spezifische DNA-Synthese (% bezogen auf Lösungsmittelkontrolle)

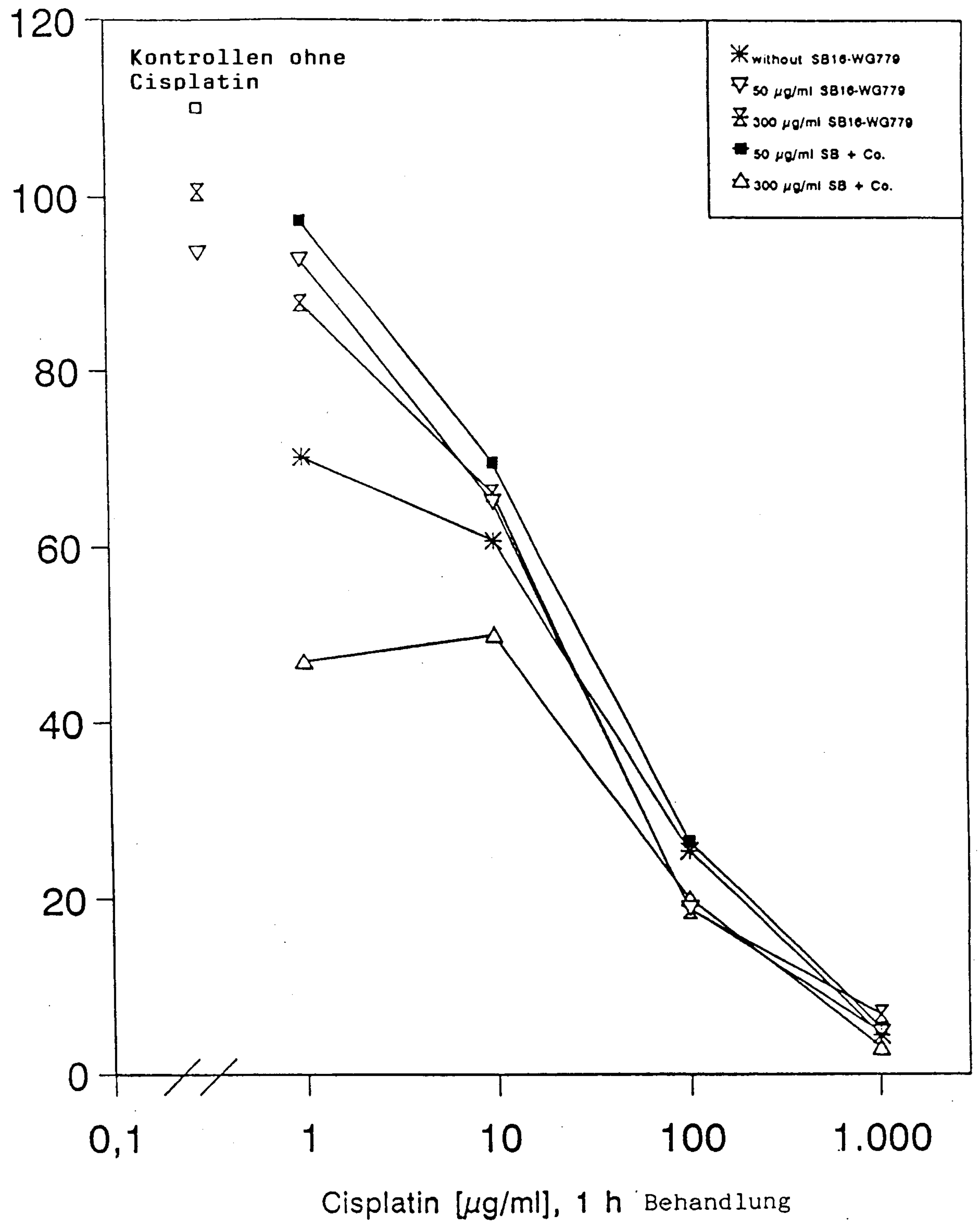


SB = Silibinin als SB16-WG779
SB16-WG779 = Silibinin-C-2',3-dihydrogensuccinat, Dinatrium (Silibinindihemisuccinat)

Figur 16
Spezifische Protein-Synthese (% bez. auf Lösungsmittelkontrolle)

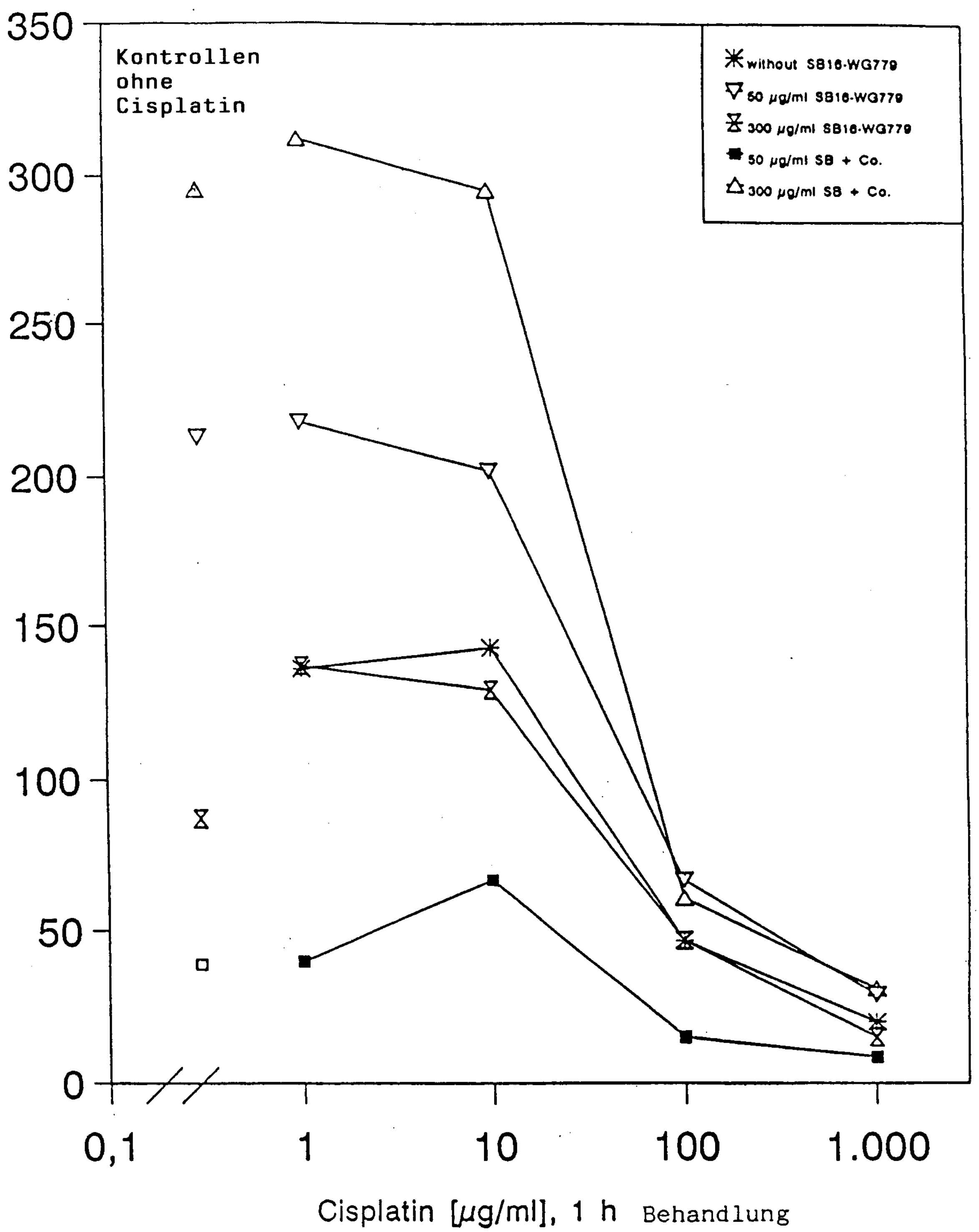


SB = Silibinin als SB16-WG779
SB16-WG779 = Silibinin-C-2',3-dihydrogensuccinat, Dinatrium (Silibinindihemisuccinat)

Europäisches Patentamt

# EUROPÄISCHER RECHERCHENBERICHT

Nummer der Anmeldung
EP 95 10 0817

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| P,A | US-A-5 336 685 (H. J. PROCHASKA ET AL)<br>* Ansprüche 1-3 * | 1-13 | A61K31/35<br>A61K31/335<br>A61K47/22 |
| A,D | EP-A-0 209 037 (INVERNI DELLA BEFFA SPA)<br>* das ganze Dokument * | 1-13 | |
| A | DATABASE BIOSIS<br>BIOSCIENCES INFORMATION SERVICE, PHILADELPHIA, PA, US<br>BA90:125188,<br>NEELAM S S ET AL 'Combination of flavoneacetic acid (FAA) with adriamycin, cisplatinum and difluoromethylornithine (DFMO) in-vitro against human colon cancer cells '<br>* Zusammenfassung *<br>& INVEST NEW DRUGS 8 (3) 1990 P.263-268, | 1-13 | |
| A | DATABASE CHEMABS<br>CHEMICAL ABSTRACTS SERVICE, COLUMBUS, OHIO, US<br>AN: 115:369,<br>WAUD, WILLIAM R. ET AL 'Antitumor drug cross-resistance in vivo in a cisplatin-resistant murine P388 leukemia'<br>* Zusammenfassung *<br>& CANCER CHEMOTHER. PHARMACOL. (1991), 27(6) 456-63, | 1-13 | RECHERCHIERTE SACHGEBIETE (Int.Cl.6)<br>A61K |
| A | GB-A-2 261 434 (INVERNI DELLA BEFFA SPA)<br>* das ganze Dokument * | 1-13 | |
| A,D | EP-A-0 422 497 (IBI-ISTITUTO BIOCHIMICO ITALIANO)<br>* das ganze Dokument * | 1-13 | |

Der vorliegende Recherchenbericht wurde für alle Patentansprüche erstellt

| Recherchenort | Abschlußdatum der Recherche | Prüfer |
|---|---|---|
| BERLIN | 19.Juni 1995 | Siatou, E |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)